(19) Europäisches Patentamt
European Patent Office
Office européen des brevets

(11) Veröffentlichungsnummer : **0 112 524**
**B1**

(12) # EUROPÄISCHE PATENTSCHRIFT

(45) Veröffentlichungstag der Patentschrift : **28.05.86**

(21) Anmeldenummer : **83112276.7**

(22) Anmeldetag : **07.12.83**

(51) Int. Cl.[4] : **C 07 C131/00**, C 07 C149/32, C 07 C161/02, C 07 C161/00, C 07 D213/64, C 07 D317/58, A 01 N 37/16, A 01 N 37/52, A 01 N 43/40, A 01 N 43/30, A 01 N 47/48

(54) **Trichloroacryloyloxim-Verbindungen.**

(30) Priorität : **17.12.82 JP 220165/82**

(43) Veröffentlichungstag der Anmeldung : **04.07.84 Patentblatt 84/27**

(45) Bekanntmachung des Hinweises auf die Patenterteilung : **28.05.86 Patentblatt 86/22**

(84) Benannte Vertragsstaaten : **BE CH DE FR GB IT LI**

(56) Entgegenhaltungen :
**US-A- 4 244 959**
**Die Akte enthält technische Angaben, die nach dem Eingang der Anmeldung eingereicht wurden und die nicht in dieser Patentschrift enthalten sind.**

(73) Patentinhaber : **NIHON TOKUSHU NOYAKU SEIZO K.K.**
**No.4, 2-Chome, Nihonbashi Honcho**
**Chuo-ku Tokyo 103 (JP)**

(72) Erfinder : **Yamada, Yasuo**
**5-15-9, Nishihirayama**
**Hino-shi Tokyo (JP)**
Erfinder : **Saito, Junichi**
**3-7-12, Osawa**
**Mitaka-shi Tokyo (JP)**
Erfinder : **Gotoh, Toshio**
**20-1-304, 5-chome, Seishin Sagamihara-shi**
**Kanagawa-ken (JP)**
Erfinder : **Katsumata, Osamu**
**438, Midori-cho**
**Hachioji-shi Tokyo (JP)**
Erfinder : **Sakawa, Shinji**
**1-14-13, Tamadaira**
**Hino-shi Tokyo (JP)**

(74) Vertreter : **Schumacher, Günter, Dr. et al**
**c/o Bayer AG Konzernverwaltung RP Patentabteilung**
**D-5090 Leverkusen 1 Bayerwerk (DE)**

**EP 0 112 524 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue Trichloroacryloyloxim-Verbindungen, ein Verfahren zu ihrer Herstellung und ihre Verwendung als landwirtschaftliche Fungizide.

Es ist bereits bekannt, daß bestimmte Oxim-Derivate wie beispielsweise 1,3-Dichloroacetonoximtrichloroacetat und 2-Chlorocyclohexanonoxim-3-chlorobutyrat fungizide Eigenschaften besitzen (vgl. die US-PSen 3 733 419 und 4 059 625). Die Herstellung anderer Oxim-Derivate wie α-(Benzoxazol-2-yl)-α-(O-chloromethylcarbonyl-oximino) acetonitril sowie ihre Verwendung als Antidot für Herbizide sind bekannt (vgl. die Europäische Patentanmeldung 0 012 158).

Weiterhin sind O-Acyl-(α-haloformaldoxim)-pyridine, wie z. B. das 2-(α-Chloroformaldoxim)-pyridin, u. a. als Fungizide beschrieben (vgl. US-A 4 244 959).

Nunmehr wurden neue Trichloroacryloyloxim-Verbindungen der allgemeinen Formel (I)

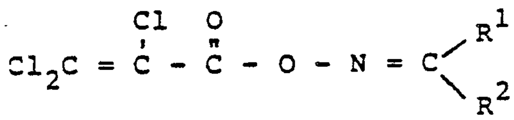 (I)

in der $R^1$ und $R^2$ jeweils für ein Wasserstoff-Atom, eine niedere Alkyl-Gruppe, eine Benzyl-Gruppe, eine Benzylthio-Gruppe, eine niedere Alkylthio-Gruppe, eine Cycloalkyl-Gruppe, ein Halogen-Atom, eine Styryl-Gruppe, eine Halogenophenylthio-Gruppe, eine Naphthyl-Gruppe oder eine Phenyl-Gruppe stehen, die gegebenenfalls substituiert sein kann durch 1 bis 5 Halogen-Atome, eine Nitro-Gruppe, eine niederen Alkyl-Gruppe, eine niederen Alkoxy-Gruppe, eine Phenoxy-Gruppe, eine Benzyloxy-Gruppe, eine niederen Alkylthio-Gruppe, eine Thiocyanato-Gruppe, eine Di-niederalkylamino-Gruppe, eine Halogenopyridyloxy-Gruppe und eine Methylendioxy-Gruppe, oder $R^1$ und $R^2$ zusammen mit dem Kohlenstoff-Atom, an das sie gebunden sind, einen aliphatischen Ring mit 5 bis 8 Kohlenstoff-Atomen bilden können, wobei dieser Ring gegebenenfalls mit 1 bis 5 Methyl-Gruppen substituiert ist.

Weiterhin wurde gefunden, daß die neuen Trichloroacryloyloxim-Verbindungen der allgemeinen Formel (I)

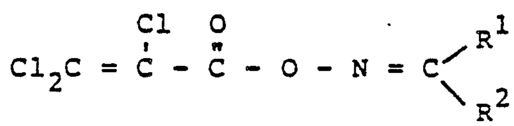 (I)

in der $R^1$ und $R^2$ jeweils für ein Wasserstoff-Atom, eine niedere Alkyl-Gruppe, eine Benzyl-Gruppe, eine Benzylthio-Gruppe, eine niedere Alkylthio-Gruppe, eine Cycloalkyl-Gruppe, ein Halogen-Atom, eine Styryl-Gruppe, eine Halogenophenylthio-Gruppe, eine Naphthyl-Gruppe oder eine Phenyl-Gruppe stehen, die gegebenenfalls substituiert sein kann durch 1 bis 5 Halogen-Atome, eine Nitro-Gruppe, eine niederen Alkyl-Gruppe, eine niederen Alkoxy-Gruppe, eine Phenoxy-Gruppe, eine Benzyloxy-Gruppe, eine niederen Alkylthio-Gruppe, eine Thiocyanato-Gruppe, eine Di-niederalkylamino-Gruppe, eine Halogenopyridyloxy-Gruppe oder eine Methylendioxy-Gruppe, oder $R^1$ und $R^2$ zusammen mit dem Kohlenstoff-Atom, an das sie gebunden sind, einen aliphatischen Ring mit 5 bis 8 Kohlenstoff-Atomen bilden können, wobei dieser Ring gegebenenfalls mit 1 bis 5 Methyl-Gruppen substituiert ist, erhalten werden durch ein Verfahren, bei dem

a) ein Acylhalogenid der allgemeinen Formel (II)

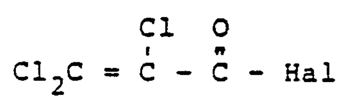 (II)

in der Hal ein Halogen-Atom bezeichnet, mit einem Oxim der allgemeinen Formel (III)

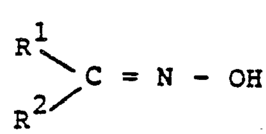 (III)

umgesetzt wird, in der $R^1$ und $R^2$ die oben angegebenen Bedeutungen haben, gegebenenfalls in Gegenwart eines Verdünnungsmittels und gegebenenfalls in Gegenwart eines säurebindenden Mittels, oder bei dem

b) Trichloroacryloylsäure-Derivate der allgemeinen Formel (IV)

$$Cl_2C = \overset{Cl}{\overset{|}{C}} - CO - OM \qquad (IV),$$

in der M ein Alkalimetall-Atom oder Wasserstoff bezeichnet, mit einem Ameisensäureesterhalogenid-Derivat der Formel (V)

$$Hal—CO—O—alk \qquad (V)$$

umgesetzt wird, in der Hal ein Halogen-Atom bezeichnet und alk eine niedere Alkyl-Gruppe bezeichnet, wodurch Verbindungen der allgemeinen Formel (VI)

$$Cl_2C = \overset{Cl}{\overset{|}{C}} - CO - O - CO - Oalk \qquad (VI)$$

erhalten werden, die in einem zweiten Schritt mit einem Oxim-Derivat der Formel (III) unter Bildung der Verbindungen der Formel (I) gemäß der vorliegenden Erfindung weiter reagieren, gegebenenfalls in Gegenwart eines Verdünnungsmittels und gegebenenfalls in Gegenwart eines säurebindenden Mittels.

Weiterhin wurde gefunden, daß die Trichloroacryloyloxim-Verbindungen der Formel (I), in denen $R^1$ und $R^2$ die im Vorstehenden genannten Bedeutungen haben, starke biologische Aktivitäten, insbesondere fungizide Eigenschaften, besitzen.

Diese fungizide Aktivität kann zweckmäßig ausgenutzt werden durch den Einsatz der Verbindungen als landwirtschaftliche Fungizide und ist besonders geeignet zur Bekämpfung der Brusone-Krankheit von Reis (rice blast).

Außerdem wurde auch gefunden, daß die Verbindungen gemäß der vorliegenden Erfindung zusätzlich zu der oben erwähnten Aktivität ein ausgezeichnetes Eindringvermögen besitzen, das ihrer Strukturcharakteristik zuzuschreiben ist, so daß sie ihre ausgeprägte Bekämpfungswirkung gegen die Brusone-Krankheit von Reis insbesondere dann zu entfalten vermögen, wenn sie auf die Oberfläche oder in das Innere des Wassers eines Reisfeldes gebracht werden und demzufolge mit Vorteil zur Einsparung landwirtschaftlicher Arbeitskraft verwendet werden können.

Weiterhin wurde gefunden, daß die Verbindungen der vorliegenden Erfindung nur geringe Toxizität gegenüber warmblütigen Tieren besitzen und in den gebräuchlichen Konzentrationen keinerlei Phytotoxizität bei Kulturpflanzen verursachen und demgemäß auch unter dem Gesichtspunkt der Sicherheit vorteilhaft einzusetzen sind.

Infolgedessen stellen die Substanzen gemäß der vorliegenden Erfindung eine Bereicherung des Standes der Technik dar.

Die Formel (I) liefert eine allgemeine Definition der Trichloroacryloyloxim-Verbindungen gemäß der vorliegenden Erfindung.

Bevorzugte Verbindungen der Formel (I) sind diejenigen, in denen $R^1$ und $R^2$ unabhängig voneinander Wasserstoff, geradkettiges oder verzweigtes Alkyl mit 1 bis 6 Kohlenstoff-Atomen, Benzyl, Benzylthio, geradkettiges oder verzweigtes Alkylthio mit 1 bis 6 Kohlenstoff-Atomen, Cycloalkyl mit 3 bis 6 Kohlenstoff-Atomen, Halogen-Atome, insbesondere Fluor, Chlor, Brom oder Iod, Styryl, Phenylthio, das mit 1 bis 5 Halogen-Atomen, insbesondere Fluor, Chlor, Brom oder Iod substituiert ist, Naphthyl oder Phenyl bezeichnen, das gegebenenfalls mit 1 bis 3 gleichen oder verschiedenen Substituenten substituiert sein kann, die ausgewählt sind aus Halogen, Nitro, niederem Alkyl oder Alkoxy, Phenoxy, Benzyloxy, niederem Alkylthio, Thiocyanato, Di-niederalkylamino, Halogenopyridyloxy oder Methylendioxy. In den vorgenannten Substituenten besitzen sämtliche Alkyl-Struktureinheiten 1 bis 6 Kohlenstoff-Atome. Oder aber $R^1$ und $R^2$ können zusammen mit dem Kohlenstoff-Atom, an das sie gebunden sind, einen aliphatischen Ring mit 5 bis 8 Kohlenstoff-Atomen bilden, der durch 1 bis 3 Methyl-Gruppen substituiert sein kann.

Besonders bevorzugte Verbindungen der Formel (I) sind diejenigen, in denen $R^1$ und $R^2$ ein Wasserstoff-Atom, Methyl, Ethyl, Propyl, Isopropyl oder n-(iso-, sec- oder tert-)Butyl, eine Benzyl-Gruppe, eine Benzylthio-Gruppe, Methylthio, Ethylthio, n-Propylthio, Isopropylthio, n-, iso-, sec- oder tert-Butylthio, Cyclopropyl, Cyclobutyl, Cyclopentyl oder Cyclohexyl, Fluoro, Chloro, Bromo oder Iodo, eine Styryl-Gruppe, eine Halogenophenylthio-Gruppe mit den gleichen Halogen-Atomen wie oben beispielhaft angeführt, eine Naphthyl-Gruppe oder eine Phenyl-Gruppe bezeichnen, die gegebenenfalls substituiert sein kann durch mindestens einen der Substituenten Fluoro, Chloro, Bromo, Iodo, Nitro, Methyl, Ethyl, n- oder iso-Propyl, n-, sec-, iso- oder tert-Butyl, Methoxy, Ethoxy, n- oder Isopropoxy, n-, sec.-, iso- oder tert.-Butoxy, Phenoxy, Benzyloxy, Methylthio, Ethylthio, n- oder Isopropylthio, n-, sec.-, iso- oder tert.-Butylthio, Thiocyanato, Dimethylamino Diethylamino, Di-n-propylamino, Di-iso-propylamino, Di-n-butylamino, Chlorpyridyloxy, Fluoropyridyloxy, Bromopyridyloxy und Methylendioxy, oder solche Verbindungen, in denen $R^1$ und $R^2$ zusammen mit dem Kohlenstoff-Atom, an das sie gebunden sind, ein Cyclopentan, Cyclohexan, Cycloheptan oder Cyclooctan bilden. Diese Ringe können durch 1 oder 2 Methyl-Gruppen substituiert sein.

Wenn beispielsweise Trichloroacryloylchlorid und Benzaldehydoxim als Ausgangssubstanzen eingesetzt werden, läßt sich der Ablauf der Reaktion bei dem Verfahren a) gemäß der vorliegenden Erfindung durch die folgende Gleichung darstellen :

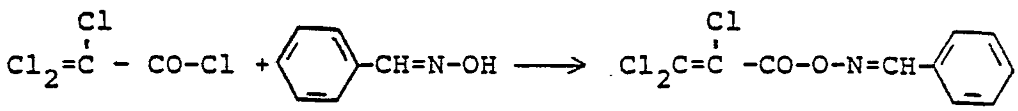

Wenn beispielsweise das Natriumsalz der Trichloroacryloylsäure, der Ethylester von Ameisensäurechlorid und Benzaldehydoxim als Ausgangssubstanzen eingesetzt werden, läßt sich der Ablauf der Reaktion bei dem Verfahren b) gemäß der vorliegenden Erfindung durch die folgende Gleichung darstellen :

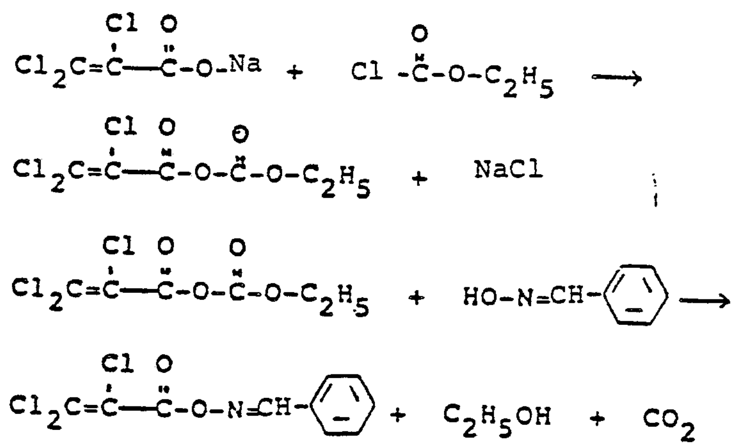

Die Acylhalogenide der Formel (II), die als Ausgangssubstanzen für das Verfahren a) gemäß der vorliegenden Erfindung benötigt werden, sind bekannt (vgl. beispielsweise die CS-PS 106 553).

Spezielle Beispiele für die Acylhalogenide der allgemeinen Formel (II) sind Trichloroacryloylchlorid und -bromid.

Die Formel (III) liefert eine allgemeine Definition der Oxime, die weiter als Ausgangssubstanzen für das Verfahren a) gemäß der vorliegenden Erfindung benötigt werden. Die meisten der Oxime der Formel (III) sind bekannt (vgl. beispielsweise die DE-PS 952 088), oder sie können mit Hilfe wohlbekannter Verfahren gewonnen werden.

Zu speziellen Beispielen für die Oxime der allgemeinen Formel (III) zählen Benzaldehydoxim, 1-Naphthaldehydoxim, 2-Chlorobenzaldehydoxim, 3-Chlorobenzaldehydoxim, 4-Chlorobenzaldehydoxim, 2-Bromobenzaldehydoxim, 2,4-Dichlorobenzaldehydoxim, 3,4-Dichlorobenzaldehydoxim, 3,5-Dichlorobenzaldehydoxim, 2,6-Dichlorobenzaldehydoxim, 4-Nitrobenzaldehydoxim, 2-Nitrobenzaldehydoxim, o-Tolualdehydoxim, p-Tolualdehydoxim, 2-Methoxybenzaldehydoxim, 2,4-Dimethoxybenzaldehydoxim, 3,4-Methylendioxybenzaldehydoxim, 3-Phenoxybenzaldehydoxim, 3-Benzyloxybenzaldehydoxim, 3-(5-Chloro-2-pyridyloxy)-benzaldehydoxim, 4-Methylthiobenzaldehydoxim, 4-Thiocyanatobenzaldehydoxim, 4-Dimethylaminobenzaldehydoxim, α-Chlorobenzaldehydoxim, α-Chloro-2-chlorobenzaldehydoxim, Benzylidenacetophenonoxim, α-4-Chlorophenylthiobenzaldehydoxim, Acetophenonoxim, Acetoxim, α-Ethylthioacetaldehydoxim, α-Benzylthioacetaldehydoxim, Phenylacetonoxim, Dicyclopropylketonoxim, Cyclopentanonoxim, Cyclohexanonoxim, Cyclooctanonoxim, 4-Methylcyclohexanonoxim und 2,5-Dimethylcyclohexanonoxim.

Die Trichloroacryloylsäure-Derivate der Formel (IV) und die Ameisensäureesterhalogenid-Derivate der Formel (V), die weiterhin als Ausgangssubstanzen für das Verfahren b) gemäß der vorliegenden Erfindung benötigt werden, sind sämtlich allgemein bekannte Verbindungen.

Die vorstehenden Verfahren können zweckmäßigerweise unter Verwendung eines Lösungsmittels oder Verdünnungsmittels durchgeführt werden. Zu diesem Zweck können sämtliche inerten Lösungsmittel und Verdünnungsmittel verwendet werden.

Beispiele für solche Lösungsmittel oder Verdünnungsmittel sind aliphatische, alicyclische und aromatische Kohlenwasserstoffe (die gegebenenfalls chloriert sein können) wie Hexan, Cyclohexan, Petrolether, Ligroin, Benzol, Toluol, Xylol, Methylenchlorid, Chloroform, Kohlenstofftetrachlorid, Ethylenchlorid, Trichloroethylen und Chlorbenzol ; Ether wie Diethylether, Methylethylether, Di-isopropylether, Dibutylether, Propylenoxid, Dioxan und Tetrahydrofuran ; Ketone wie Aceton, Methylethylketon, Methylisopropylketon und Methylisobutylketon ; Nitrile wie Acetonitril, Propionitril und Acrylnitril ; Ester wie Ethylacetat und Amylacetat ; Säureamide wie Dimethylformamid und Dimethylacetamid ; Sulfone und Sulfoxide wie Dimethylsulfoxid und Sulfolan ; sowie Basen wie Pyridin.

Die Reaktionen gemäß der vorliegenden Erfindung können in Gegenwart eines säurebindenden Mittels durchgeführt werden. Beispiele für solche säurebindenden Mittel sind die Alkalimetall-Hydroxide, Carbonate, Hydrogencarbonate und Alkoholate, die im allgemeinen verwendet werden, sowie tertiäre Amine wie Triethylamin, Diethylanilin und Pyridin.

Das Verfahren gemäß der vorliegenden Erfindung kann bei einer Temperatur innerhalb eines weiten Bereichs durchgeführt werden. Beispielsweise kann es bei einer Temperatur zwischen etwa —20 °C und dem Siedepunkt der Mischung durchgeführt werden, günstigerweise zwischen etwa 0 °C und etwa 100 °C. Zweckmäßigerweise wird die Reaktion unter atmosphärischem Druck durchgeführt. Jedoch ist es auch möglich, unter erhöhtem oder vermindertem Druck zu arbeiten.

Die aktiven Verbindungen gemäß der vorliegenden Erfindung zeigen eine starke biologische Wirksamkeit und können in der Praxis zur Bekämpfung unerwünschter Mikroorganismen eingesetzt werden. Die aktiven Verbindungen eignen sich für die Verwendung als Fungizide.

Die Verbindungen gemäß der vorliegenden Erfindung besitzen ein breites fungizides Spektrum und können in wirksamer Weise gegen verschiedene Pflanzenkrankheiten eingesetzt werden, die beispielsweise durch Archimycetes, Phycomycetes, Ascomycetes, Basidiomycetes und Fungi imperfecti sowie durch Bakterien verursacht werden.

Als Pflanzenschutzmittel können die aktiven Verbindungen gemäß der vorliegenden Erfindung mit besonders gutem Erfolg als landwirtschaftliche Fungizide verwendet werden, insbesondere zur Bekämpfung der Brusone-Krankheit von Reis.

Als landwirtschaftliches Fungizid können die Verbindungen gemäß der vorliegenden Erfindung unmittelbar nach Verdünnen mit Wasser oder aber in Form verschiedener Formulierungen eingesetzt werden, wie sie unter Verwendung landwirtschaftlich unbedenklicher Hilfsstoffe durch Verfahren gewonnen werden, die bei der praktischen Herstellung von Agrochemikalien allgemein angewandt werden, wobei diese Präparate entweder unmittelbar oder nach dem Verdünnen mit Wasser auf die gewünschte Konzentration zur Anwendung gelangen können.

Beispiele für die landwirtschaftlich unbedenklichen Hilfsstoffe, auf die hier Bezug genommen wird, sind Verdünnungsmittel (Lösungsmittel, Streckmittel, Träger), oberflächenaktive Mittel (Lösungsvermittler, Emulgatoren, Dispergiermittel, Netzmittel), Stabilisatoren, Haftmittel, Aerosol-Treibmittel und synergistische Mittel.

Beispiele für die Lösungsmittel sind Wasser und organische Lösungsmittel, beispielsweise Kohlenwasserstoffe [z. B. n-Hexan, Petrolether, Naphtha, Erdöl-Fraktionen (z. B. Paraffinwachse, Kerosin, Leichtöle, Mittelöle, Schweröle), Benzol, Toluol und Xylole], halogenierte Kohlenwasserstoffe (z. B. Methylenchlorid, Kohlenstofftetrachlorid, Trichloroethylen, Ethylenchlorid, Ethylendibromid, Chlorbenzol und Chloroform), Alkohole (z. B. Methylalkohol, Ethylalkohol, Propylalkohol und Ethylenglycol), Ether (z. B. Diethylether, Ethylenoxid und Dioxan), Alkohol-ether (z. B. Ethylenglycol-monomethylether), Ketone (z. B. Aceton und Isophoron), Ester (z. B. Ethylacetat und Amylacetat), Amide (z. B. Dimethylformamid und Dimethylacetamid) und Sulfoxide (z. B. Dimethylsulfoxid).

Beispiele für die Streckmittel oder Träger umfassen anorganische Pulver, beispielsweise Löschkalk, Magnesiumkalk, Gips, Calciumcarbonat, Siliciumdioxid, Perlit, Bimsstein, Calcit, Diatomeenerde, amorphes Siliciumdioxid, Aluminiumoxid, Zeolithe und Tonminerale (z. B. Pyrophyllit, Talkum, Montmorillonit, Beidellit, Vermikulit, Kaolinit und Glimmer), pflanzliche Pulver wie beispielsweise Getreidepulver, Stärkearten, verarbeitete Stärkearten, Zucker, Glucose und zerkleinerte Stengel von Pflanzen, sowie Pulver aus synthetischen Harzen wie Phenol-Harzen, Harnstoff-Harzen und Vinylchlorid-Harzen.

Beispiele für oberflächenaktive Mittel umfassen anionische oberflächenaktive Mittel wie Alkylsulfonsäureester (z. B. Natriumlaurylsulfat), Arylsulfonsäuren (z. B. Alkylarylsulfonsäure-Salze und Natriumalkylnaphthalinsulfonate), Bernsteinsäure-Salze und Salze von Schwefelsäureestern von Polyethylenglycol-alkylarylethern, kationische oberflächenaktive Mittel wie Alkylamine (z. B. Laurylamin, Stearyltrimethylammoniumchlorid und Alkyldimethylbenzylammoniumchlorid) und Polyoxyethylenalkylamine, nicht-ionische oberflächenaktive Mittel wie Polyoxyethylenglycolether (z. B. Polyoxyethylenalkylarylether und deren Kondensationsprodukte), Polyoxyethylenglycolester (z. B. Polyoxyethylenfettsäureester) und Ester mehrwertiger Alkohole (z. B. Polyoxyethylensorbitan-monolaurat) sowie amphotere oberflächenaktive Mittel.

Beispiele für andere Hilfsstoffe umfassen Stabilisatoren, Haftmittel (z. B. landwirtschaftliche Seifen, Casein-Kalk, Natriumalginat, Polyvinylalkohol, Haftmittel vom Vinylacetat-Typ und Acryl-Haftmittel), Aerosol-Treibmittel (z. B. Trichlorofluoromethan, Dichlorofluoromethan, 1,2,2-Trichloro-1,1,2-trifluoroethan, Chlorbenzol, verflüssigtes Erdgas und niedere Ether), die Verbrennung steuernde Mittel für Räucherpräprate (z. B. Nitrite, Zink-Pulver und Dicyandiamid), Sauerstoff abgebende Mittel (z. B. Chlorate und Bichromate), wirkungsverlängernde Mittel, Dispersions-Stabilisatoren [z. B. Casein, Tragant, Carboxymethylcellulose (CMC) und Polyvinylalkohol (PVA)] und synergistische Mittel.

Die Verbindungen der vorliegenden Erfindung können mittels der allgemein auf dem Gebiet der Agrochemikalien gebräuchlichen Verfahren zu verschiedenen Präparaten formuliert werden. Beispiele für solche Anwendungsformen sind emulgierbare Konzentrate, Öl-Präparate, benetzbare Pulver, lösliche Pulver, Suspensionen, Stäubemittel, Granulat, pulvrige Präparate, Räuchermittel, Tabletten, Aerosole, Pasten und Kapseln.

Die landwirtschaftlichen Fungizide gemäß der vorliegenden Erfindung können etwa 0,1 bis etwa

95 Gew.-%, vorzugsweise etwa 0,5 bis etwa 90 Gew.-% des vorerwähnten aktiven Wirkstoffes enthalten.

Für den praktischen Gebrauch beträgt die geeignete Menge der aktiven Verbindung in den vorgenannten Mitteln in ihren verschiedenen Formen und gebrauchsfertigen Präparaten beispielsweise etwa 0,000 1 bis etwa 20 Gew.-%, vorzugsweise etwa 0,005 bis etwa 10 Gew.-%.

Der Gehalt des Wirkstoffs kann in geeigneter Weise je nach der Form des Präparats oder Mittels, dem Verfahren, Zweck, der Zeit und dem Ort seiner Anwendung, dem Zustand des Auftretens einer Pflanzenkrankheit etc. variiert werden.

Erforderlichenfalls können die Verbindungen gemäß der vorliegenden Erfindung weiterhin auch in Kombination mit anderen Agrochemikalien verwendet werden, beispielsweise mit Insektiziden, anderen Fungiziden, Mitiziden, Nematiziden, Anti-Virus-Mitteln, Herbiziden, Pflanzen-Wachstumsregulatoren und Lockstoffen [Organophosphat-Verbindungen, Carbamat-Verbindungen, Dithio- (oder thiol)carbamat-Verbindungen, organischen Chlor-Verbindungen, Dinitro-Verbindungen, organischen Schwefel- oder Metall-Verbindungen, Antibiotika, substituierten Diphenylether-Verbindungen, Harnstoff-Verbindungen und Triazin-Verbindungen] und/oder Düngemitteln.

Die den im Vorstehenden bezeichneten Wirkstoff enthaltenen verschiedenartigen Mittel und gebrauchsfertigen Präparate können mittels verschiedener Verfahren zur Anwendung gebracht werden, wie sie allgemein für das Aufbringen von Agrochemikalien gebraüchlich sind, beispielsweise durch Sprühen (Versprühen von Flüssigkeiten, Vernebeln, Zerstäuben, Stäuben, Streuen von Granulat, Wasser-Oberflächenbehandlung, Gießen etc.) Räuchern, Bodenbehandlung (Vermischen mit dem Boden, Streuen, Bedampfen, Gießen etc.), Oberflächenbehandlung (Beschichten, Bändern, Pulverbeschichten, Bedecken etc.) und Eintauchen. Sie können auch mittels des sogenannten Ultra-Low-Volume-Sprühverfahrens aufgebracht werden. Nach diesem Verfahren kann der Wirkstoff sogar in einer Konzentration von 100 % zur Anwendung gelangen.

Die Aufwandmengen pro Flächeneinheit betragen beispielsweise etwa 0,03 bis etwa 10 kg/ha, vorzugsweise etwa 0,3 bis etwa 6 kg/ha. In besonders gelagerten Fällen können oder sollten sogar die Aufwandmengen außerhalb des angegebenen Bereichs liegen.

Gemäß der vorliegenden Erfindung kann ein landwirtschaftliches Fungizid zur Verfügung gestellt werden, das als Wirkstoff die Verbindung der allgemeinen Formel (I) sowie ein Verdünnungsmittel (ein Lösungsmittel und/oder ein Streckmittel und/oder einen Träger) und/oder ein oberflächenaktives Mittel und, falls weiterhin erforderlich, einen Stabilisator, ein Haftmittel, ein synergistisches Mittel etc. enthält.

Die vorliegende Erfindung macht weiterhin ein Verfahren zur Bekämpfung von Pflanzenkrankheiten verfügbar, das darin besteht, daß eine Verbindung der allgemeinen Formel (I) allein oder in Form einer Mischung mit einem Verdünnungsmittel (einem Lösungsmittel und/oder einem Streckmittel und/oder einem Träger) und/oder einem oberflächenaktiven Mittel und, falls weiterhin erforderlich, einem Stabilisator, einem Haftmittel, einem synergistischen Mittel etc. auf einen pathogenen Pilz und/oder dessen Lebensraum und den Ort des Auftretens der Pflanzenkrankheit aufgebracht wird.

Die vorliegende Erfindung wird durch die folgenden Beispiele näher erläutert. Es sei jedoch darauf hingewiesen, daß die vorliegende Erfindung nicht allein auf diese speziellen Beispiele beschränkt ist.

## Herstellungsbeispiele

### Beispiel 1

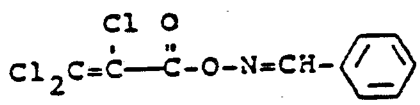

Benzaldehydoxim (30 g) und 26 g Triethylamin wurden in 400 ml Toluol gelöst, und 100 ml einer Toluol-Lösung von 40 g Trichloroacryloylchlorid wurden bei einer Temperatur unter 20 °C zugesetzt.

Die Mischung wurde 2 h bei 50 °C zur Reaktion gebracht. Das entstandene Triethylaminhydrochlorid wurde durch Filtration abgetrennt, und die organische Schicht wurde mit Wasser gewaschen und getrocknet. Das Toluol wurde abgedampft, und der Rückstand wurde aus Diethylether/n-Hexan umkristallisiert, wonach 58 g an Benzaldehydtrichloroacryloyloxim in Form weißer Kristalle erhalten wurden. Das Produkt besitzt einen Schmelzpunkt von 79-80 °C.

Tabelle 1 enthält Verbindungen gemäß der vorliegenden Erfindung, die im wesentlichen nach der gleichen Methode wie vorstehend beschrieben synthetisiert wurden :

(Siehe Tabelle 1 Seite 7 ff.)

Tabelle 1

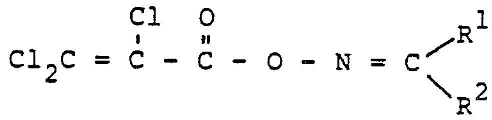

(I)

| Beispiel Nr. | 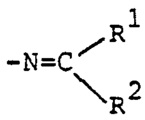 | Physikal. Konstante |
|---|---|---|
| 2 | 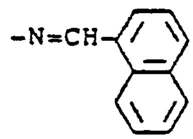 | Schmp. 62-65°C |
| 3 | 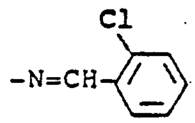 | Schmp. 75-76.5°C |
| 4 | 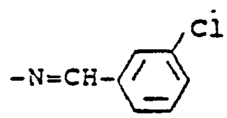 | Schmp. 69-71.5°C |
| 5 | 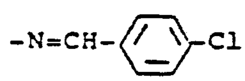 | Schmp. 100-102°C |
| 6 | 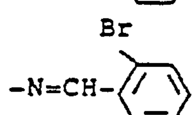 | Schmp. 79-80°C |
| 7 | 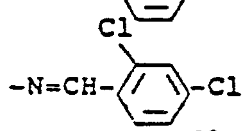 | Schmp. 102-104°C |
| 8 | 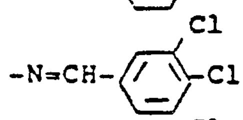 | Schmp. 91-93°C |
| 9 | 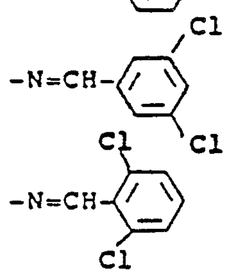 | Schmp. 100-102°C |
| 10 | | Schmp. 91-92°C |
| 11 | 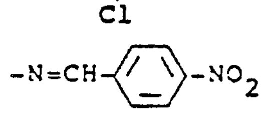 | Schmp. 118-120°C |
| 12 | 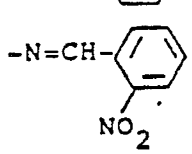 | Schmp. 81-85°C |
| 13 | 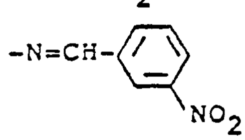 | Schmp. 101-102°C |

Tabelle 1 (Fortsetzung)

| Bei-spiel Nr. | 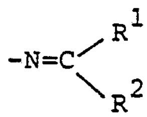 | Physikal. Konstante |
|---|---|---|
| 14 | 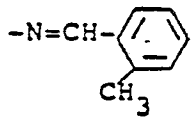 | Schmp. 62-63°C |
| 15 | 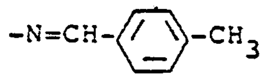 | Schmp. 78-79.5°C |
| 16 | 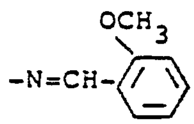 | Schmp. 76.5-77.5°C |
| 17 | 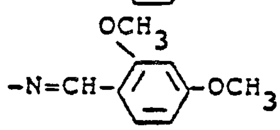 | IR:$\nu_{C=O}$ 1745 $cm^{-1}$ (Nujol) |
| 18 | 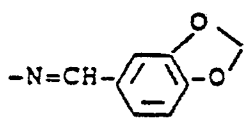 | Schmp. 113-114.5°C |
| 19 | 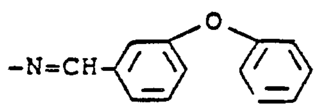 | IR:$\nu_{C=O}$ 1745 $cm^{-1}$ (unverdünnt) |
| 20 | 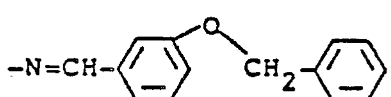 | Schmp. 95-96°C |
| 21 | 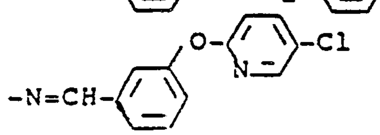 | Schmp. 93-97°C |
| 22 | 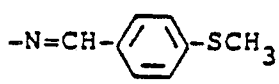 | Schmp. 105-106°C |
| 23 | 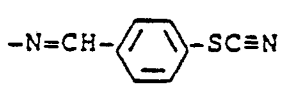 | Schmp. 136-137°C |
| 24 | 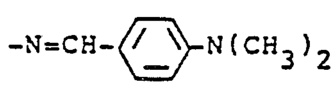 | Schmp. 110-111°C |
| 25 | 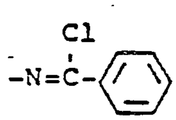 | Schmp. 43-45°C |
| 26 | 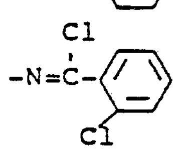 | Schmp. 58-60°C |

Tabelle 1 (Fortsetzung)

| Bei- spiel Nr. | 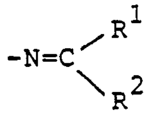 | Physikal. Konstante |
|---|---|---|
| 27 | 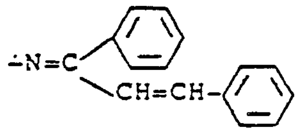 | Schmp. 188-189°C |
| 28 | 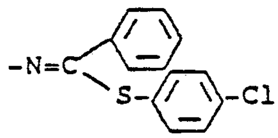 | Schmp. 91.5-93°C |
| 29 | 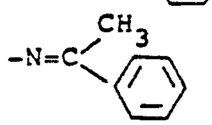 | Schmp. 62-63°C |
| 30 | 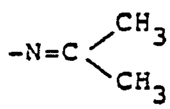 | IR: $\nu_{C=O}$ 1750 $cm^{-1}$ (unverdünnt) |
| 31 | 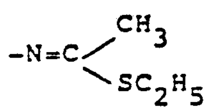 | IR: $\nu_{C=O}$ 1750 $cm^{-1}$ (unverdünnt) |
| 32 | 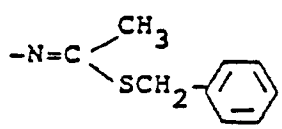 | Schmp. 74-74.5°C |
| 33 | 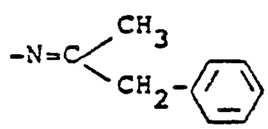 | $n_D^{20}$ 1.5445 |
| 34 | 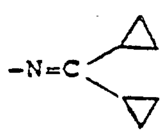 | $n_D^{20}$ 1.5440 |
| 35 | 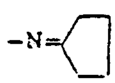 | $n_D^{20}$ 1.5478 |
| 36 | 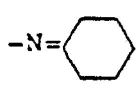 | Schmp. 44-44.5°C |
| 37 | 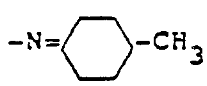 | $n_D^{20}$ 1.5242 |
| 38 | 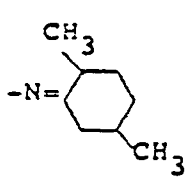 | $n_D^{20}$ 1.5195 |

Tabelle 1 (Fortsetzung)

| Beispiel Nr. | 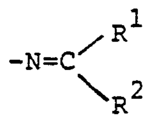 | Physikal. Konstante |
|---|---|---|
| 39 | 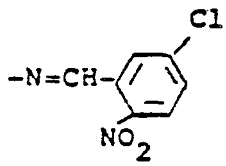 | Schmp. 114-117 °C |
| 40 | 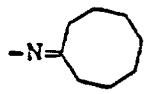 | $n_D^{20}$ 1.5392 |

Formulierungsbeispiele

a) Benetzbares Pulver

15 Teile der Verbindung Nr. 33 der Erfindung, 80 Teile eines Gemisches (1 : 5) aus Weißruß (wasserhaltigem amorphen Siliciumdioxid-Pulver) und Tonpulver, 2 Teile Natriumalkylbenzolsulfonat und 3 Teile Natriumalkylnaphthalinsulfonat/Formaldehyd-Kondensat werden pulverisiert und zu einem benetzbaren Pulver vermischt. Dieses wird mit Wasser verdünnt und auf einen pathogenen Pilz und/oder seinen Lebensraum und den Ort, an den eine Pflanzenkrankheit auftritt, aufgesprüht.

b) Emulgierbares Konzentrat

30 Teile der Verbindung Nr. 5 der Erfindung, 55 Teile Xylol, 8 Teile Polyoxyethylen-alkylphenylether und 7 Teile Calciumalkylbenzolsulfonat werden unter Rühren miteinander vermischt, wodurch ein emulgierbares Konzentrat hergestellt wird. Dieses wird mit Wasser verdünnt und auf einen pathogenen Pilz und/oder seinen Lebensraum und den Ort, an dem eine Pflanzenkrankheit auftritt, aufgesprüht.

c) Stäubemittel

2 Teile der Verbindung Nr. 10 der Erfindung und 98 Teile Tonpulver werden pulverisiert und gemischt, wodurch ein Stäubemittel hergestellt wird. Dieses wird über einem pathogenen Pilz und/oder seinen Lebensraum und den Ort, an dem eine Pflanzenkrankheit auftritt, ausgestreut.

d) Stäubemittel

Die Verbindung Nr. 6 der Erfindung (1,5 Teile), 0,5 Teile Isopropylhydrogenphosphat und 98 Teile Tonpulver werden pulverisiert und gemischt, wodurch ein Stäubemittel hergestellt wird. Dieses wird über einem pathogenen Pilz und/oder seinen Lebensraum und den Ort, an dem eine Pflanzenkrankheit auftritt, ausgestreut.

e) Granulat

Wasser (25 Teile) wird zu einer Mischung aus 10 Teilen der Verbindung Nr. 16 der Erfindung, 30 Teilen Bentonit (Montmorillonit), 58 Teilen Talkum und 2 Teilen Ligninsulfonat zugesetzt, und das Gemisch wird gut geknetet. Die Mischung wird mittels eines Extruder-Granulators zu einem Granulat mit einer Korngröße von 0,43 bis 2,0 mm (10 bis 40 mesh) verarbeitet und bei 40 °C bis 50 °C getrocknet, wodurch ein Granulat hergestellt wird. Das Granulat wird über einem pathogenen Pilz und/oder seinen Lebensraum und den Ort, an dem eine Pflanzenkrankheit auftritt, ausgestreut.

f) Granulat

Ein Drehmischer wird mit 95 Teilen Tonmineral-Teilchen mit einer Teilchengrößen-Verteilung im Bereich von 0,2 bis 2 mm beschickt, und unter Drehen des Mischers werden 5 Gew.-Teile der Verbindung Nr. 1 der Erfindung, gelöst in einem organischen Lösungsmittel auf die Tonmineral-Teilchen aufgesprüht,

so daß diese gleichmäßig benetzt werden. Die Tonmineral-Teilchen werden dann bei 40 °C bis 50 °C getrocknet, wodurch ein Granulat gebildet wird. Das Granulat wird über einem pathogenen Pilz und/oder seinen Lebensraum und den Ort, an dem eine Pflanzenkrankheit auftritt, ausgestreut.

## Verwendungsbeispiele

### Test-Beispiel A

Test der Wirksamkeit gegen die Brusone-Krankheit von Reis durch Wasser-Oberflächen-Anwendung :

Herstellung des Test-Präparates :

| | |
|---|---|
| Wirkstoff : | 50 Gew.-Teile ; |
| Träger : | 45 Gew.-Teile einer Mischung (1 : 5) aus Diatomeenerde und Kaolin ; |
| Emulgator : | 5 Gew.-Teile Polyoxyethylenalkylphenylether. |

Der Wirkstoff, der Träger und der Emulgator wurden in den angegebenen Mengen miteinander vermischt, wodurch ein benetzbares Pulver gebildet wurde. Eine vorher festgelegte Menge des benetzbaren Pulvers wurde mit Wasser verdünnt, wodurch ein Test-Präparat gebildet wurde.

Test-Verfahren A

Reispflanzen (Varietät : Asahi) wurden in überfluteten Porzellan-Töpfen mit einem Durchmesser von 12 cm, jeweils drei Stecklinge pro Topf, kultiviert. Im Stadium des Auftretens neuer Triebe wurde das Test-Präparat, das mit vorher festgelegter Konzentration in der oben angegebenen Weise hergestellt worden war, in den angegebenen Mengen auf die Wasseroberfläche gegeben, so daß es nicht in direkte Berührung mit den unterirdischen Teilen der Reispflanzen spritzte. Vier Tage später wurde eine Suspension von Sporen des Pilzes der Brusone-Krankheit (Piricularia oryzae) auf die Reispflanzen gesprüht, um sie mit dem Pilz zu beimpfen. Die Pflanzen wurden 24 h in einem Inkubator gehalten, in dem eine Temperatur von 23 °C bis 25 °C und eine relative Luftfeuchtigkeit von 100 % aufrechterhalten wurden. Danach wurden sie in ein Gewächshaus aus Glas mit einer Temperatur von 20 °C bis 28 °C überführt. Sieben Tage nach der Beimpfung wurde der Grad der Erkrankung pro Topf aufgrund des folgenden Bewertungsmaßstabs bestimmt, und der nachstehend definierte Bekämpfungs-Index wurde berechnet.

| Grad der Erkrankung | Fläche der Verletzungen (%) |
|---|---|
| 0 | 0 |
| 0,5 | 2 oder weniger |
| 1 | 3 bis 5 |
| 2 | 6 bis 10 |
| 3 | 11 bis 20 |
| 4 | 21 bis 40 |
| 5 | 41 oder mehr |

$$\text{Bekämpfungs-Index (\%)} = \frac{\text{Grad der Erkrankung einer unbehandelten Fläche} - \text{Grad der Erkrankung einer behandelten Fläche}}{\text{Grad der Erkrankung einer unbehandelten Fläche}} \times 100$$

Die nachstehend bezeichnete Substanz wird in den folgenden Verwendungsbeispielen als Vergleichssubstanz verwendet :

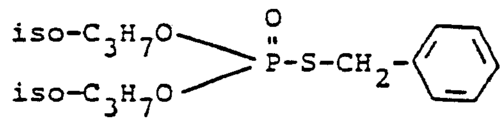

(A)

S-Benzyl-O,O-diisopropyl-phosphorothioat (Kitazin P).

In diesem Test beweisen die Verbindungen der Herstellungsbeispiele Nr. 1, 5, 10, 16 und 36 gegenüber der Vergleichsverbindung nach dem Stand der Technik eine deutlich überlegene Wirksamkeit, wie aus der Tabelle A zu ersehen ist.

Tabelle A

| Beispiel Nr. | Wirkstoff-Konzentration ($g/m^2$) | Bekämpfungs-Index (%) | Phyto-toxizität |
|---|---|---|---|
| 1 | 0,2 | 100 | - |
| 5 | 0,2 | 100 | - |
| 10 | 0,2 | 100 | - |
| 16 | 0,2 | 100 | - |
| 36 | 0,2 | 100 | - |
| A (Vergleich) | 0,51 | 80 | - |

Anmerkungen zu Tabelle A:

1) Das Symbol « — » in der Spalte « Phytotoxizität » der Tabelle gibt an, daß keine Phytotoxizität auftrat.

2) A: Kitazin P, 17 % Granulat (im Handel erhältlich).

Die anderen, nicht in dem Beispiel A gezeigten Verbindungen gemäß der vorliegenden Erfindung zeigten eine hervorragende Bekämpfungswirkung (einen Bekämpfungs-Index von nahezu 100 %) bei Wirkstoff-Konzentrationen von 0,2 $g/m^2$ und 0,8 $g/m^2$ ohne jegliche Phytotoxizität gegenüber Reispflanzen.

Test-Beispiel B

Test der Wirksamkeit gegen die Brusone-Krankheit von Reis durch Anwendung auf Stengel und Laub:

Test-Verfahren B

Reispflanzen (Varietät: Asahi) wurden in unglasierten Töpfen mit einem Durchmesser von 12 cm kultiviert. Im 3- bis 4-Blatt-Stadium wurde eine Verdünnung einer Test-Verbindung, die wie in Beispiel A in vorher festgelegter Konzentration hergestellt worden war, auf die Töpfe in einer Aufwandmenge von 50 ml für jeweils 3 Töpfe aufgesprüht. Am nächsten Tage wurde eine Suspension von Sporen des Pilzes der Brusone-Krankheit (Piricularia oryzae), der künstlich kultiviert worden war, zweimal auf die Reispflanzen gesprüht. Die Pflanzen wurden in einer Kammer bei einer Temperatur von 25 °C und einer relativen Luftfeuchtigkeit von 100 % gehalten, um eine Infektion zu bewirken. Die Pflanzen wurden 7 Tage nach der Beimpfung in der gleichen Weise wie in Beispiel A geprüft, und der Bekämpfungs-Index (%) wurde berechnet.

In diesem Test beweisen die Verbindungen der Herstellungsbeispiele Nr. 3, 5, 6, 8, 9, 10, 14, 15, 16, 23, 25, 29, 33, 34, 35 und 37 gegenüber der Vergleichsverbindung nach dem Stand der Technik eine deutlich überlegene Wirksamkeit, wie aus der Tabelle B zu ersehen ist.

Tabelle B

| Beispiel Nr. | Wirkstoff-Konzentration (ppm) | Bekämpfungs-Index (%) | Phyto-toxizität |
|---|---|---|---|
| 3 | 500 | 92 | - |
| 5 | 500 | 100 | - |
| 6 | 500 | 100 | - |

(Fortsetzung)

| Beispiel Nr. | Wirkstoff-Konzentration (ppm) | Bekämpfungs-Index (%) | Phyto-toxizität |
|---|---|---|---|
| 8 | 500 | 90 | - |
| 9 | 500 | 92 | - |
| 10 | 500 | 100 | - |
| 14 | 500 | 100 | - |
| 15 | 500 | 94 | - |
| 16 | 500 | 100 | - |
| 23 | 500 | 100 | - |
| 25 | 500 | 92 | - |
| 29 | 500 | 96 | - |
| 33 | 500 | 100 | - |
| 34 | 500 | 95 | - |
| 35 | 500 | 100 | - |
| 37 | 500 | 100 | - |
| Kontrolle: | | | |
| A' | 500 | 78 | - |

Anmerkungen zu Tabelle B :
1) Die Bezeichnung in der Spalte « Phytotoxizität » ist die gleiche wie in der Tabelle A.
2) A' : Kitazin P, 48 % emulgierbares Konzentrat (im Handel erhältlich).

## Patentansprüche

1. Trichloroacryloyloxim-Verbindungen der allgemeinen Formel (I)

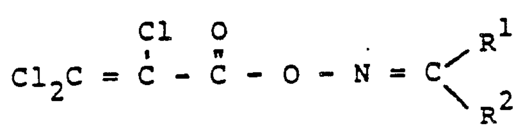

(I)

in der $R^1$ und $R^2$ jeweils für ein Wasserstoff-Atom, eine niedere Alkyl-Gruppe, eine Benzyl-Gruppe, eine Benzylthio-Gruppe, eine niedere Alkylthio-Gruppe, eine Cycloalkyl-Gruppe, ein Halogen-Atom, eine Styryl-Gruppe, eine Halogenophenylthio-Gruppe, eine Naphthyl-Gruppe oder eine Phenyl-Gruppe stehen, die gegebenenfalls substituiert sein kann durch 1 bis 5 Halogen-Atome, eine Nitro-Gruppe, eine niederen Alkyl-Gruppe, niedere Alkoxy-Gruppe, eine Phenoxy-Gruppe, eine Benzyloxy-Gruppe, eine niedere Alkylthio-Gruppe, eine Thiocyanato-Gruppe, eine Di-niederalkylamino-Gruppe, eine Halogenopyridyloxy-Gruppe und eine Methylendioxy-Gruppe oder $R^1$ und $R^2$ zusammen mit dem Kohlenstoff-Atom, an das sie gebunden sind, einen aliphatischen Ring bilden können, wobei dieser Ring gegebenenfalls mit 1 bis 5 Methyl-Gruppen substituiert ist.

2. Trichloroacryloyloxim-Verbindungen nach Anspruch 1, dadurch gekennzeichnet, daß in der Formel (I) $R^1$ und $R^2$ unabhängig voneinander Wasserstoff, geradkettiges oder verzweigtes Alkyl mit 1 bis 6 Kohlenstoff-Atomen, Benzyl, Benzylthio, geradkettiges oder verzweigtes Alkylthio mit 1 bis 6 Kohlenstoff-Atomen, Cycloalkyl mit 3 bis 6 Kohlenstoff-Atomen, Halogen-Atome, Styryl, Phenylthio, das mit 1 bis 5 Halogen-Atomen substituiert ist, Naphthyl oder Phenyl bezeichnen, das gegebenenfalls mit 1 bis 3 gleichen oder verschiedenen Halogenen, Nitro, niederem Alkyl oder Alkoxy, Phenoxy, Benzyloxy, niederem Alkylthio, Thiocyanato, Di-niederalkylamino, Halogenopyridyloxy oder Methylendioxy substituiert sein kann, wobei in den vorgenannten Substituenten sämtliche Alkyl-Struktureinheiten 1 bis 6

Kohlenstoff-Atome besitzen, oder aber $R^1$ und $R^2$ zusammen mit dem Kohlenstoff-Atom, an das sie gebunden sind, einen aliphatischen Ring mit 5 bis 8 Kohlenstoff-Atomen bilden können, der durch 1 bis 3 Methyl-Gruppen substituiert sein kann.

3. Trichloroacryloyloxim-Verbindungen nach Anspruch 1, dadurch gekennzeichnet, daß in der Formel (I) $R^1$ und $R^2$ ein Wasserstoff-Atom, Methyl, Ethyl, Propyl, Isopropyl oder n-(iso-, sec- oder tert-) Butyl, eine Benzyl-Gruppe, eine Benzylthio-Gruppe, Methylthio, Ethylthio, n-Propylthio, Isopropylthio, n-, iso-, sec- oder tert-Butylthio, Cyclopropyl, Cyclobutyl, Cyclopentyl oder Cyclohexyl, Fluoro, Chloro, Bromo oder Iodo, eine Styryl-Gruppe, eine Halogenophenylthio-Gruppe mit den gleichen Halogen-Atomen wie oben beispielhaft angeführt, eine Naphthyl-Gruppe oder eine Phenyl-Gruppe bezeichnen, die gegebenenfalls ein- oder zweifach substituiert sein kann durch Fluoro, Chloro, Bromo, Iodo, Nitro, Methyl, Ethyl, n- oder iso-Propyl, n-, sec-, iso- oder tert-Butyl, Methoxy, Ethoxy, n- oder Isopropoxy, n-, sec-, iso- oder tert.-Butoxy, Phenoxy, Benzyloxy, Methylthio, Ethylthio, n- oder iso-Propylthio, n-, sec-, iso- oder tert.-Butylthio, Thiocyanato, Dimethylamino, Diethylamino, Di-n-propylamino, Di-iso-propylamino, Di-n-butylamino, Chloropyridyloxy, Fluoro-pyridyloxy, Bromopyridyloxy und Methylendioxy, oder $R^1$ und $R^2$ zusammen mit dem Kohlenstoff-Atom, an das sie gebunden sind, ein Cyclopentan, Cyclohexan, Cycloheptan oder Cyclooctan bilden, wobei diese Ringe durch 1 oder 2 Methyl-Gruppen substituiert sein können.

4. Verfahren zur Herstellung von Trichloroacryloyloxim-Verbindungen der allgemeinen Formel (I)

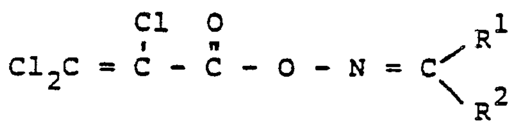

(I)

in der $R^1$ und $R^2$ jeweils für ein Wasserstoff-Atom, eine niedere Alkyl-Gruppe, eine Benzyl-Gruppe, eine Benzylthio-Gruppe, eine niedere Alkylthio-Gruppe, eine Cycloalkyl-Gruppe, ein Halogen-Atom, eine Styryl-Gruppe, eine Halogenophenylthio-Gruppe, eine Naphthyl-Gruppe oder eine Phenyl-Gruppe stehen, die gegebenenfalls substituiert sein kann durch 1 bis 5 Halogen-Atome, eine Nitro-Gruppe, eine niedere Alkyl-Gruppe, eine niedere Alkoxy-Gruppe, eine Phenoxy-Gruppe, eine Benzyloxy-Gruppe, eine niedere Alkylthio-Gruppe, eine Thiocyanato-Gruppe, eine Di-niederalkylamino-Gruppe, eine Halogenopyridyloxy-Gruppe und eine Methylendioxy-Gruppe oder $R^1$ und $R^2$ zusammen mit dem Kohlenstoff-Atom, an das sie gebunden sind, einen aliphatischen Ring mit 5 bis 8 Kohlenstoff-Atomen bilden können, wobei dieser Ring gegebenenfalls mit 1 bis 5 Methyl-Gruppen substituiert ist, dadurch gekennzeichnet, daß

a) ein Acylhalogenid der allgemeinen Formel (II)

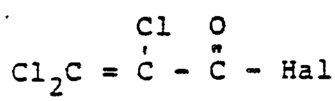

(II)

in der Hal ein Halogen-Atom bezeichnet, mit einem Oxim der allgemeinen Formel (III)

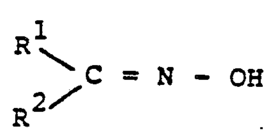

(III)

umgesetzt wird, in der $R^1$ und $R^2$ die oben angegebenen Bedeutungen haben, gegebenenfalls in Gegenwart eines Verdünnungsmittels und gegebenenfalls in Gegenwart eines säurebindenden Mittels, oder

b) Trichloroacryloylsäure-Derivate der allgemeinen Formel (IV)

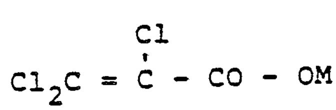

(IV)

in der M ein Alkalimetall-Atom oder Wasserstoff bezeichnet, mit einem Ameisensäureesterhalogenid-Derivat der Formel (V)

Hal—CO—O—alk (V)

umgesetzt wird, in der Hal ein Halogen-Atom bezeichnet und alk eine niedere Alkyl-Gruppe bezeichnet, wodurch Verbindungen der allgemeinen Formel (VI)

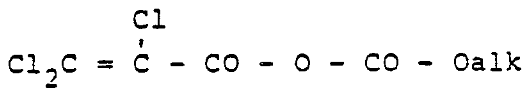

(VI)

erhalten werden, die in einem zweiten Schritt mit einem Oxim-Derivat der Formel (III) unter Bildung der Verbindungen der Formel (I) gemäß der vorliegenden Erfindung weiter reagieren, gegebenenfalls in Gegenwart eines Verdünnungsmittels und gegebenenfalls in Gegenwart eines säurebindenden Mittels.

5. Mittel zur Pilzbekämpfung, dadurch gekennzeichnet, daß sie wenigstens eine substituierte Trichloroacryloyloxim-Verbindung der Formel (I) nach Ansprüchen 1 oder 4 enthalten.

6. Verwendung von Trichloroacryloyloxim-Verbindungen der Formel (I) nach Ansprüchen 1 oder 4 zur Bekämpfung von Pilzen.

7. Verfahren zur Pilzbekämpfung, dadurch gekennzeichnet, daß man Trichloroacryloyloxim-Verbindungen der Formel (I) nach Ansprüchen 1 oder 4 auf Pilze und/oder ihre Umgebung einwirken läßt.

8. Verfahren zur Herstellung von Mitteln zur Pilzbekämpfung, dadurch gekennzeichnet, daß Trichloroacryloyloxim-Verbindungen der Formel (I) nach Ansprüchen 1 oder 4 mit Streckmitteln und/oder oberflächenaktiven Mitteln vermischt werden.

**Claims**

1. Trichloroacryloyl oxime compounds of the general formula (I)

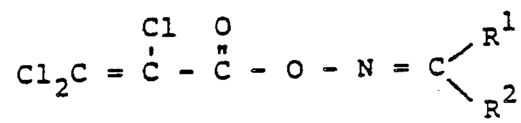

(I)

in which $R^1$ and $R^2$ each represent a hydrogen atom, a lower alkyl group, a benzyl group, a benzylthio group, a lower alkylthio group, a cycloalkyl group, a halogen atom, a styryl group, a halogenophenylthio group, a naphthyl group or a phenyl group which can optionally be substituted by 1 to 5 halogen atoms, a nitro group, a lower alkyl group, a lower alkoxy group, a phenoxy group, a benzyloxy group, a lower alkylthio group, a thiocyanato group, a di-lower alkylamino group, a halogenopyridyloxy group and a methylenedioxy group or $R^1$ and $R^2$, together with the carbon atom to which they are bonded, can form an aliphatic ring, this ring being optionally substituted by 1 to 5 methyl groups.

2. Trichloroacryloyl oxime compounds according to claim 1, characterised in that in the formula (I) $R^1$ and $R^2$ independently of one another designate hydrogen, straight-chain or branched alkyl with 1 to 6 carbon atoms, benzyl, benzylthio, straight-chain or branched alkylthio with 1 to 6 carbon atoms, cycloalkyl with 3 to 6 carbon atoms, halogen atoms, styryl, phenylthio, which is substituted by 1 to 5 halogen atoms, naphthyl or phenyl, which can optionally be substituted by 1 to 3 identical or different halogen atoms, nitro, lower alkyl or alkoxy, phenoxy, benzyloxy, lower alkylthio, thiocyanato, di-lower alkylamino, halogenopyridyloxy or methylenedioxy, all of the alkyl structural units in the aforementioned substituents having 1 to 6 carbon atoms, or $R^1$ and $R^2$, together with the carbon atom to which they are bonded, can form an aliphatic ring with 5 to 8 carbon atoms which can be substituted by 1 to 3 methyl groups.

3. Trichloroacryloyl oxime compounds according to claim 1, characterised in that, in the formula (I), $R^1$ and $R^2$ designate a hydrogen atom, methyl, ethyl, propyl, isopropyl or n-(iso-, sec- or tert-)butyl, a benzyl group, a benzylthio group, methylthio, ethylthio, n-propylthio, isopropylthio, n-, iso-, sec- or tert-butylthio, cyclopropyl, cyclobutyl, cyclopentyl or cyclohexyl, fluoro, chloro, bromo or iodo, a styryl group, a halogenophenylthio group with the same halogen atoms as exemplified above, a naphthyl group or a phenyl group which can optionally be mono- or disubstituted by fluoro, chloro, bromo, iodo, nitro, methyl, ethyl, n- or iso-propyl, n-, sec-, iso- or tert-butyl, methoxy, ethoxy, n- or isopropoxy, n-, sec.-, iso or tert.-butoxy, phenoxy, benzyloxy, methylthio, ethylthio, n- or iso-propylthio, n-, sec.-, iso- or tert.-butylthio, thiocyanato, dimethylamino, diethylamino, di-n-propylamino, di-iso-propylamino, di-n-butylamino, chloropyridyloxy, fluoropyridyloxy, bromopyridyloxy and methylenedioxy, or $R^1$ and $R^2$, together with the carbon atom to which they are bonded, form a cyclopentane, cyclohexane, cycloheptane or cyclooctane, it being possible for these rings to be substituted by 1 or 2 methyl groups.

4. Process for the preparation of trichloroacryloyl oxime compounds of the general formula (I)

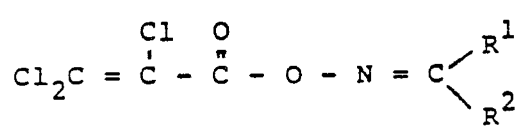

(I)

in which $R^1$ and $R^2$ each represent a hydrogen atom, a lower alkyl group, a benzyl group, a benzylthio group, a lower alkylthio group, a cycloalkyl group, a halogen atom, a styryl group, a halogenophenylthio

group, a naphthyl group or a phenyl group, which can optionally be substituted by 1 to 5 halogen atoms, a nitro group, a lower alkyl group, a lower alkoxy group, a phenoxy group, a benzyloxy group, a lower alkylthio group, a thiocyanato group, a di-lower alkylamino group, a halogenopyridyloxy group and a methylenedioxy group or $R^1$ and $R^2$, together with the carbon atom to which they are bonded, can form an aliphatic ring with 5 to 8 carbon atoms, this ring optionally being substituted by 1 to 5 methyl groups, characterised in that

a) an acyl halide of the general formula (II)

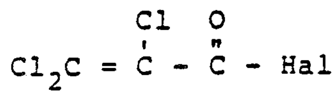

(II)

in which Hal designates a halogen atom, is reacted with an oxime of the general formula (III)

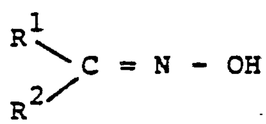

(III)

in which $R^1$ and $R^2$ have the meanings given above, if appropriate in the presence of a diluent and if appropriate in the presence of an acid-binding agent, or

b) trichloroacryloyl acid derivatives of the general formula (IV)

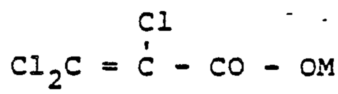

(IV)

in which M designates an alkali metal atom or hydrogen, is reacted with a formic acid ester halide derivative of the formula (V)

Hal—CO—O—alk (V)

in which Hal designates a halogen atom and alk designates a lower alkyl group, whereby compounds of the general formula (VI)

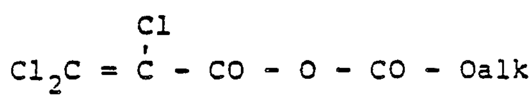

(VI)

are obtained which react further, in a second step, with an oxime derivative of the formula (III) to form the compounds of the formula (I) according to the present invention, if appropriate in the presence of a diluent and if appropriate in the presence of an acid-binding agent.

5. Agents for combating fungi, characterised in that they contain at least one substituted trichloroacryloyl oxime compound of the formula (I) according to claims 1 or 4.

6. Use of trichloroacryloyl oxime compounds of the formula (I) according to claims 1 or 4 for cambating fungi.

7. Method of combating fungi, characterised in that trichloroacryloyl oxime compounds of the formula (I) according to claims 1 or 4 are allowed to act on fungi and/or their surroundings.

8. Process for the preparation of agents for combating pests, characterised in that trichloroacryloyl oxime compounds of the formula (I) according to Claims 1 or 4 are mixed with extenders and/or surface-active agents.

**Revendications**

1. Composés de trichloracryloyloximes de formule générale (I) :

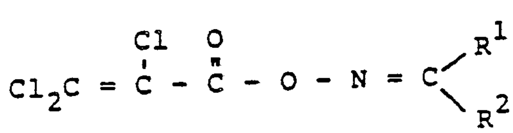

(I)

dans laquelle $R^1$ et $R^2$ représentent chacun un atome d'hydrogène, un groupe alkyle inférieur, un groupe benzyle, un groupe benzylthio, un groupe alkyl inférieur-thio, un groupe cycloalkyle, un atome

d'halogène, un groupe styryle, un groupe halogénophénylthio, un groupe naphtyle ou un groupe phényle qui peut éventuellement être substitué par 1 à 5 atomes d'halogènes, par un groupe nitro, par un groupe alkyle inférieur, par un groupe alcoxy inférieur, par un groupe phénoxy, par un groupe benzyloxy, par un groupe alkyl inférieur-thio, par un groupe thiocyanato, par un groupe di-alkyl inférieur-amino, par un groupe halogénopyridyloxy et par un groupe méthylène-dioxy, ou $R^1$ et $R^2$, ensemble avec l'atome de carbone auquel ils sont reliés, peuvent former un noyau aliphatique, ce noyau étant éventuellement substitué par 1 à 5 groupes méthyle.

2. Composés de trichloracryloyloximes selon la revendication 1, caractérisés en ce que, dans la formule (I), $R^1$ et $R^2$ représentent chacun indépendamment l'un de l'autre, un atome d'hydrogène, un groupe alkyle à chaîne droite ou ramifiée contenant 1 à 6 atomes de carbone, un groupe benzyle, un groupe benzylthio, un groupe alkylthio à chaîne droite ou ramifiée contenant 1 à 6 atomes de carbone, un groupe cycloalkyle contenant 3 à 6 atomes de carbone, un atome d'halogène, un groupe styryle, un groupe phénylthio qui est substitué par 1 à 5 atomes d'halogènes, un groupe naphtyle ou un groupe phényle qui peut éventuellement être substitué par 1 à 3 atomes d'halogènes identiques ou différents, par un groupe nitro, par un groupe alkyle ou alcoxy inférieur, par un groupe phénoxy, par un groupe benzyloxy, par un groupe alkyl inférieur-thio, par un groupe thiocyanato, par un groupe di-alkyl inférieur-amino, par un groupe halogénopyridyloxy ou par un groupe méthylène-dioxy, tous les motifs structuraux alkyle des substituants précités comportant 1 à 6 atomes de carbone, ou $R^1$ et $R^2$, ensemble avec l'atome de carbone auquel ils sont reliés, peuvent former un noyau aliphatique contenant 5 à 8 atomes de carbone, ce noyau pouvant être substitué par un à trois groupes méthyle.

3. Composés de trichloracryloyloximes selon la revendication 1, caractérisés en ce que, dans la formule (I), $R^1$ et $R^2$ représentent chacun un atome d'hydrogène, un groupe méthyle, un groupe éthyle, un groupe propyle, un groupe isopropyle ou un groupe n-(iso-, sec- ou tert-)butyle, un groupe benzyle, un groupe benzylthio, un groupe méthylthio, un groupe éthylthio, un groupe n-propylthio, un groupe isopropylthio, un groupe n-, iso-, sec- ou tert-butylthio, un groupe cyclopropyle, un groupe cyclobutyle, un groupe cyclopentyle ou un groupe cyclohexyle, un atome de fluor, un atome de chlore, un atome de brome ou un atome d'iode, un groupe styryle, un groupe halogénophénylthio comportant les mêmes atomes d'halogènes que ceux mentionnés ci-dessus à titre d'exemple, un groupe naphtyle ou un groupe phényle qui peut éventuellement être substitué une ou deux fois par un atome de fluor, par un atome de chlore, par un atome de brome, par un atome d'iode, par un groupe nitro, par un groupe méthyle, par un groupe éthyle, par un groupe n- ou iso-propyle, par un groupe n-, sec-, iso- ou tert-butyle, par un groupe méthoxy, par un groupe éthoxy, par un groupe n- ou iso-propoxy, par un groupe n-, sec-, iso- ou tert-butoxy, par un groupe phénoxy, par un groupe benzyloxy, par un groupe méthylthio, par un groupe éthylthio, par un groupe n- ou iso-propylthio, par un groupe n-, sec-, iso- ou tert-butylthio, par un groupe thiocyanato, par un groupe diméthylamino, par un groupe diéthylamino, par un groupe di-n-propylamino, par un groupe di-iso-propylamino, par un groupe di-n-butylamino, par un groupe chloropyridyloxy, par un groupe fluoropyridyloxy, par un groupe bromopyridyloxy et par un groupe méthylène-dioxy, ou $R^1$ et $R^2$, ensemble avec l'atome de carbone auquel ils sont reliés, forment un noyau cyclopentane, un noyau cyclohexane, un groupe cycloheptane ou un noyau cyclo-octane, ces noyaux pouvant être substitués par un ou deux groupes méthyle.

4. Procédé de préparation de composés de trichloracryloyloximes de formule générale (I) :

$$Cl_2C = \overset{\displaystyle Cl}{\overset{|}{C}} - \overset{\displaystyle O}{\overset{\|}{C}} - O - N = C\begin{matrix} \diagup R^1 \\ \diagdown R^2 \end{matrix} \qquad (I)$$

dans laquelle $R^1$ et $R^2$ représentent chacun un atome d'hydrogène, un groupe alkyle inférieur, un groupe benzyle, un groupe benzylthio, un groupe alkyl inférieur-thio, un groupe cycloalkyle, un atome d'halogène, un groupe styryle, un groupe halogénophénylthio, un groupe naphtyle ou un groupe phényle qui peut éventuellement être substitué par 1 à 5 atomes d'halogènes, par un groupe nitro, par un groupe alkyle inférieur, par un groupe alcoxy inférieur, par un groupe phénoxy, par un groupe benzyloxy, par un groupe alkyl inférieur-thio, par un groupe thiocyanato, par un groupe di-alkyl inférieur-amino, par un groupe halogénopyridyloxy et par un groupe méthylène-dioxy, ou $R^1$ et $R^2$, ensemble avec l'atome de carbone auquel ils sont reliés, peuvent former un noyau aliphatique contenant 5 à 8 atomes de carbone, ce noyau étant éventuellement substitué par 1 à 5 groupes méthyle, caractérisé en ce que :

a) on fait réagir un halogénure d'acyle de formule générale (II) :

$$Cl_2C = \overset{\displaystyle Cl}{\overset{|}{C}} - \overset{\displaystyle O}{\overset{\|}{C}} - Hal \qquad (II)$$

dans laquelle Hal représente un atome d'halogène, avec une oxime de formule générale (III) :

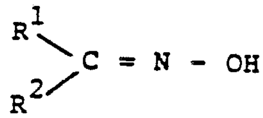 (III)

dans laquelle $R^1$ et $R^2$ ont les significations indiquées ci-dessus, éventuellement en présence d'un diluant et éventuellement en présence d'un agent fixateur d'acide, ou

b) on fait réagir des dérivés d'acide trichloracryloylique de formule générale (IV) :

$$Cl_2C = \overset{Cl}{\overset{|}{C}} - CO - OM \qquad (IV)$$

dans laquelle M représente un atome de métal alcalin ou l'hydrogène, avec un dérivé d'halogénure d'ester d'acide formique de formule (V) :

$$Hal—CO—O—alk \qquad (V)$$

dans laquelle Hal représente un atome d'halogène et alk représente un groupe alkyle inférieur, pour obtenir ainsi des composés de formule générale (VI) :

$$Cl_2C = \overset{Cl}{\overset{|}{C}} - CO - O - CO - Oalk \qquad (VI)$$

qui, dans une deuxième étape, sont soumis à une réaction complémentaire avec un dérivé d'oxime de formule (III) avec formation des composés de formule (I) selon la présente invention, éventuellement en présence d'un diluant et éventuellement en présence d'un agent fixateur d'acide.

5. Agents en vue de combattre les champignons, caractérisés en ce qu'ils contiennent au moins un composé de trichloracryloyloxime substitué de formule (I) selon les revendications 1 ou 4.

6. Utilisation de composés de trichloracryloyloximes de formule (I) selon les revendications 1 ou 4 en vue de combattre les champignons.

7. Procédé en vue de combattre les champignons, caractérisé en ce qu'on fait agir des composés de trichloracryloyloximes de formule (I) selon les revendications 1 ou 4 sur des champignons et/ou leur environnement.

8. Procédé de préparation d'agents en vue de combattre les champignons, caractérisé en ce qu'on mélange des composés de trichloracryloyloximes de formule (I) selon les revendications 1 ou 4 avec des agents délayants et/ou des agents tensio-actifs.